# EUROPEAN PATENT APPLICATION

(11) **EP 3 552 643 A1**
(43) Date of publication of application: **16.10.2019**
(21) Application number: 18178126.1
(22) Date of filing: 15.06.2018
(51) Int. Cl.: A61M 15/00, A24F 7/00

(54) **INFRARED TEMPERATURE CONTROL SYSTEM OF ELECTRONIC HEATING DEVICE AND CONTROL METHOD THEREOF**

(30) Priority: 13.04.2018 CN 201810331434
(71) Applicant: Shenzhen Hangsen Star Technology Co., Ltd., Shenzhen, Guangdong 518112 (CN)
(72) Inventor: YAO, Haofeng, Shenzhen, Guangdong 518112 (CN); ZHANG, Shipeng, Shenzhen, Guangdong 518112 (CN); MA, Fangshuo, Shenzhen, Guangdong 518112 (CN)
(74) Representative: Poskett, Oliver James

(57) **Abstract**

The invention relates to an infrared temperature control system of an electronic heating device and a control method thereof. The system comprises a control unit, an infrared detection unit, a heating unit and a heating pipe (20) with detection hole; and the detection unit is used to emit a detection signal, and to detect the conditions of the heating pipe, and to judge whether a cigarette is inserted into a heating pipe; and he infrared detection unit feeds back an input signal to the control unit, and the control unit drives the heating unit to start and adjust the heating temperature of the heating unit, and the temperature is adjusted by control unit according to the insertion condition of the cigarette to save the energy of the battery, so that the electronic heating device is more similar to the burning process of the cigarette.

## Description

### Technical Field

The invention relates to the electronic heating device manufacturing field, especially for an infrared temperature control system of an electronic heating device and control method thereof.

### Background Art

With the improvement of the people's living standards, they gradually realized the harm of smoking on the human body, and thus created electronic cigarettes. The low-temperature non-combustible baking appliance, ie, the electronic heating device, is a new type of cigarette smoking device, which is popular because the working temperature is far less than the temperature of direct burning of the cigarette.

The existing low-temperature non-combustible roasting tobacco adopts a heating tube or a heating wire, and the cigarettes on the market are mainly divided into two types, one is an ordinary cigarette, the shape is a long cylinder, one is a special cigarette, and the shape is a short cylinder, but Existing devices for flue-cured tobacco cannot automatically recognize the two types of cigarettes, and they cannot be used together, nor can they be turned off in time according to the extraction of cigarettes, resulting in waste of battery energy.

Therefore, it is necessary to design a new low-temperature control system to detect whether or not a cigarette is inserted in the heating device, adjust the temperature according to the insertion condition of the cigarette, and save battery energy.

### Summary of the Invention

The invention relates to an infrared temperature control system of an electronic heating device and a control method thereof. The system comprises a control unit, an infrared detection unit, a heating unit and a heating pipe with detection hole; and the detection unit is used to emit a detection signal, and to detect the conditions of the heating pipe, and to judge whether a cigarette is inserted into a heating pipe; and he infrared detection unit feeds back an input signal to the control unit, and the control unit drives the heating unit to start and adjust the heating temperature of the heating unit, and the temperature is adjusted by control unit according to the insertion condition of the cigarette to save the energy of the battery, so that the electronic heating device is more similar to the burning process of the cigarette, that is, it is burned from long to short to enhance the user's taste.

Based on the above defects, the purpose of the invention is to provide an infrared temperature control system of electronic heating device and control method thereof.

An infrared temperature control system of an electronic heating device comprising a control unit, an Infrared detection unit, and a heating pipe with at least two detection holes located on the same vertical line, wherein:
the infrared detection unit is configured to emit a detection signal and is configured to detect the conditions of the heating pipe through the detection hole ,and is further configured to judge whether there is a cigarette in the heating pipe and to form an input signal;
the control unit is configured to output an adjustment signal to the heating unit according to the received input signal; and
the heating unit is configured to start heating and configured to adjust the heating temperature of the heating pipe according to the adjustment signal.

Its further technical solution is that he infrared detecting unit comprises an infrared sensor and a detection drive module, wherein:
the infrared sensor is configured to detect the conditions of the heating pipe through the detection hole, and is configured to form a detection signal; and
the detection drive module is configured to form an input signal according to the detection signal.

Its further technical solution is that the detection drive module comprises a switch module connected to the receiving diode D1 of the infrared sensor. When the receiving diode D1 of the infrared sensor receives the signal of cigarette inserted, and the signal drives the switch module to turn on and to form an input signal to the control unit, and the heating unit is driven.

Its further technical solution is that the switch module comprises a first switching element Q6 and a second switching element Q5, wherein the control pin of the first switching element Q6 is connected to the anode of the receiving diode D1,and the cathode of the receiving diode D1 is connective to the external power source BT+; and the output pin of the first switching element Q6 is connected to the control terminal of the second switching element Q5 is connected; and the output pin of the second switching element Q5 is connected to the control unit.

Its further technical solution is that the control unit comprises a control chip U4.

Its further technical solution is that the heating unit comprises a heating drive module and a detection module, wherein:
the heating drive module is configured to receive a control signal and drive an external heating conductor to heat; and
the detection module is configured to obtain the current and the voltage of the heating drive module during work and is configured to feedback the signal to the control unit to adjust the heating temperature of the heating drive module.

Its further technical solution is that the heating drive module comprises a driving chip Q1 and a switching element Q2; wherein according to the received control signal the switching element Q2 is configured to control the driving chip Q1 by turning on or off, and the detection module comprises a current sampling chip U1.

Its further technical solution is that the infrared temperature control system comprises a charging unit, and according to an external power source the charging unit is configured to charge the power supply units connected with it.

Its further technical solution is that the infrared temperature control system comprises a display unit, and the display unit is configured to display the current state of the electric heating device under the control of the control unit.

The invention also provides the infrared temperature control method using the electronic heating device, comprises:
the infrared detection unit emits an infrared signal to detect the internal condition of the heating pipe through the detection hole of the heating pipe, and determines whether a cigarette is inserted into the heating pipe and forming an input signal;
the control unit receiving the input signal and outputting the adjustment signal to the heating unit; and
the heating unit adjusting the heating temperature of the heating pipe according to the adjustment signal.

The beneficial effect of the present invention, as the following:
The infrared temperature control system of the electronic heating device detects whether a cigarette is inserted into a heating pipe through the infrared detection unit and a heating pipe. The infrared detection unit feeds back an input signal to the control unit, and the control unit drives the heating unit to start and adjust the heating temperature of the heating unit, and the temperature is adjusted by control unit according to the insertion condition of the cigarette to save the energy of the battery, so that the electronic heating device is more similar to the burning process of the cigarette, that is, it is burned from long to short to enhance the user's taste.

### Description of Drawings

FIG.1 is the structural block diagram of the infrared temperature control system of the electronic heating device for an embodiment.
FIG.2 is the shape configuration diagram of the infrared detection unit for an embodiment.
FIG.3 is the circuit schematic of the infrared detection unit for an embodiment.
FIG.4 is the circuit schematic of the control unit for an embodiment.
FIG.5 is the circuit schematic of the heating unit for an embodiment.
FIG.6 is the circuit schematic of the charging unit for an embodiment;
FIG.7 is the circuit schematic of the display unit for an embodiment.

### Detailed Description of the Invention

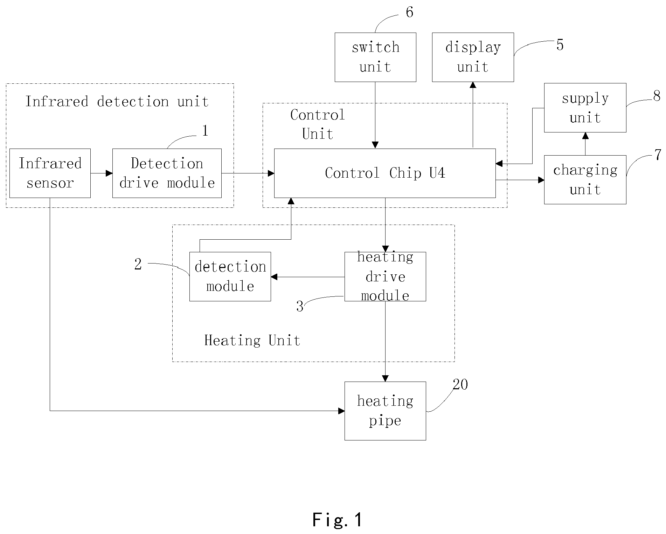

As shown in Fig.1 to Fig.7, The infrared temperature control system of the electronic heating device provided by this embodiment can be used in an electronic heating device with relatively low temperature and non-combustible baking tobacco or cigarette, so as to detect whether a cigarette is inserted into the electronic heating device, and to adjust the temperature of the heating pipe according to the insertion condition of the cigarette, and to save battery energy, so as to make the electronic heating device more similar to the cigarette burning process, that is, to from long to short burning, and to enhance the user's taste.

As shown in Fig.1, in this embodiment, an infrared temperature control system of an electronic heating device comprises a control unit, an Infrared detection unit, and a heating pipe 20 with at least two detection holes 21 located on the same vertical line, wherein:
the infrared detection unit is configured to emit a detection signal and is configured to detect the conditions of the heating pipe 20 through the detection holes 21, and is configured to judge whether there is a cigarette in the heating pipe 20 and to form an input signal;
the control unit is configured to output an adjustment signal to the heating unit according to the input signal; and
the heating unit is configured to start heating and configured to adjust the heating temperature of the heating pipe according to the adjustment signal.

After the cigarette is inserted into the heating tube 20, the infrared signal emitted by the infrared detection unit can detect the presence of the cigarette, and the infrared detection unit sends an input signal to the control unit. The control unit sends an instruction to drive the heating unit, and then the heating unit heats the heating pipe 20, and the baked the cigarette in the heating tube 20 emits a cigarette gas.

The number of heating units may be two or more. These heating units are respectively disposed in different segment areas of the heating pipe, and each heating unit corresponds to an infrared detection unit. Correspondingly, these detection holes 21 are arranged along the same vertical line on the heating tube 20. In this way, the infrared detection unit can judge whether the cigarette placed in the heating pipe 20 is a long-smoke bombarding or a short-smocking tantalum cigarette according to the reflection signal, So that the control unit controls the heating unit at the different positions to heat and to adjust the heating temperature.

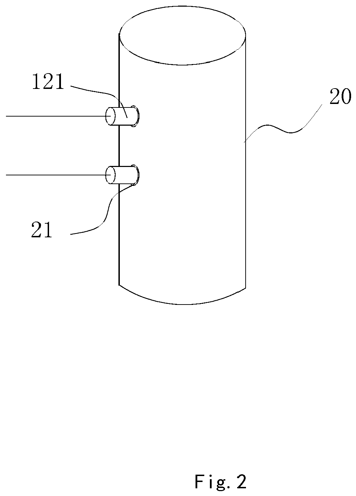

As shown in Fig.2, the infrared detection unit detects the insertion position of the cigarette to determine whether there is a cigarette inserted or the long or short state of the inserted cigarette, and feeds back different detection signals to the control unit, and the control unit sends different heating instructions to the heating unit, then the heating unit heats the heating pipe locally or heats up in stages as a whole. In this way, the electronic heating device can be adapted to special short cigarettes as well as ordinary cigarettes, so that it is more similar to the cigarette burning process, that is, it is burned from long to short in order to enhance the user's taste.

Further, the infrared detection unit comprises an infrared sensor 12 and a detection drive module 1. The infrared sensor 12 comprises the detection probes 121 inserted into the detection holes 21 disposed on the heating pipe 20. According to the detected state of heating pipe 20, the detection probe 121 is configured to form a detection signal. The detection drive module 1 is configured to form an input signal based on the detection signal sent from the infrared sensor 12, and the detection drive module 1 sends the input signal to the control unit.

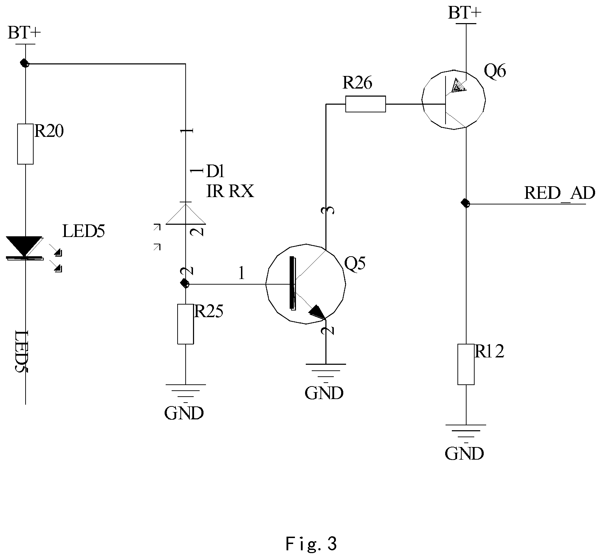

As shown in Fig 3, the detection drive module 1 comprises a switch module connected to the receiving diode D1 of the infrared sensor 12. When the receiving diode D1 detects that a cigarette is inserted into the heating pipe 20, the receiving diode D1 outputs a detection signal to the switch module; and the switch module works under the action of the detection signal, and the switch module forms an input signal. The detection drive module is i configured to form an input signal according to the detection signal, which is transited the control unit an input signal, and the control unit drive the heating unit to heat according to the received input signal.

Preferably, the switch module comprises a first switching element Q6 and a second switching element Q5, wherein the control terminal of the first switching element Q6 is connected to the anode of the receiving diode D1, and the cathode of the receiving diode D1 is connective to the external power source BT+. The output terminal of the first switching element Q6 is connected to the control terminal of the second switching element Q5; the output terminal of the second switching element Q5 is connected to the control unit.

Specifically, the detection drive module 1 comprises a first pull-down resistor R25, a second pull-down resistor R12, and a third voltage divider resistor R26. Wherein, the both ends of the first pull-down resistor R25 are respectively connected the control pin of the second switching element Q5 and the ground; and the both ends of the second pull-down resistor R12 are respectively connected the output pin of the first switching element Q6 and the ground. The both ends of the third voltage divider resistor R26 are respectively connected to the control leg of the first switching element Q6 and the output pin of the second switching element Q5; the external power source BT+ is electrically connected to the anode of the infrared diode LED5 through the current limiting resistor R20, and the cathode of the infrared diode LED5 is connected to the control unit; the connection point of the power source BT+ and the current limiting resistor R20 is electrically connected to the negative electrode of the receiving diode D1, and the anode of the receiving diode D1 is electrically connected to the control pin of the second switching element Q5.

After the receiving diode D1 receives the infrared signal, the receiving diode D1 turns on. In this time, the level of the control pin of the second switching transistor Q5 is high level, and the second switching element Q5 is turned on, so that the first switching element Q6 is also turned on, and the RED_AD level of the detection position is high level, and the control unit can assume that the cigarette is not inserted into the heating pipe 20. If the receiving diode D1 does not receive an infrared signal, the receiving diode D1 is not turned on. In this time, the level of the control pin of the second switching element Q5 is low level, the second switching element Q5 is not turned on, and then the first switching element Q6 is not turned on. The RED_AD level of the detection position is low, and the control unit can determine that the cigarette is inserted into the heating pipe 20.

The second switching element Q5 is a PNP transistor or a P-MOS transistor. The first switching element Q6 is an NPN transistor or an N-MOS transistor.

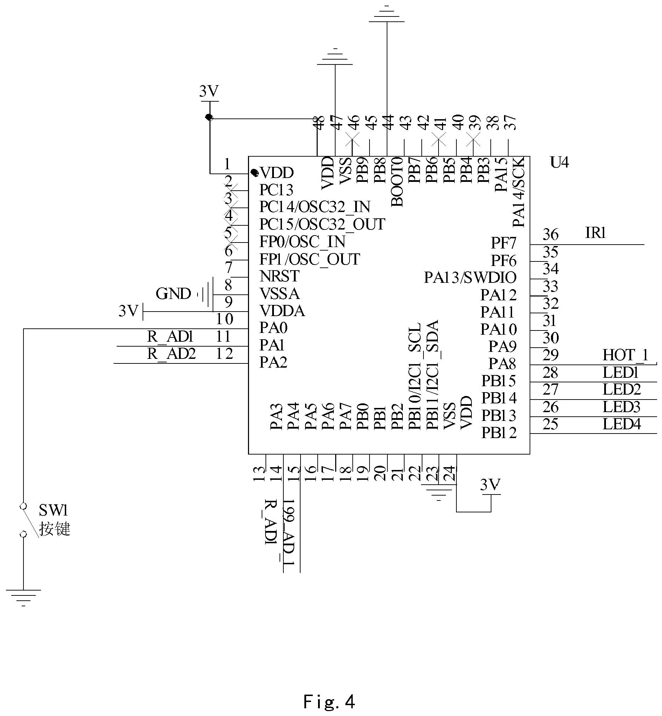

Specifically, as shown in Fig.4, in this embodiment, the above-mentioned control unit comprises a control chip U4. The model of the control chip U4 is SMT32F030CBT6. In other embodiments, the above control chip U4 may also be a chip of other SMT series.

Further, the above-mentioned heating unit comprises a heating drive module 3 and a detection module 2. The heating drive module 3 is configured to receive a control signal and is configured to drive the heating pipe 20 that is externally connected to the heating pipe 20 to heat. The detection module 2 is configured to obtain a current and a voltage of the heating drive module 3 during operation and is configured to form an input signal, and detection module 2 is configured to output the input signal to the control chip U4 of the control unit, and according to the received input signal the control chip U4 is configured to adjust the heating temperature of the drive module 3.

In this embodiment, the heating pipe 20 is a heating wire. In other embodiments, the heating pipe 20 is a combination of a heating wire and a heating tube.

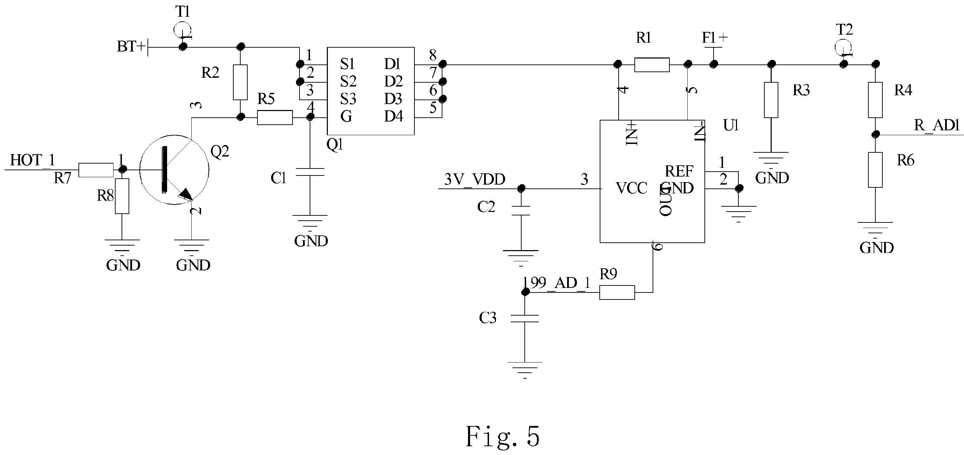

As shown in Fig.5, The above-mentioned heating drive module 3 comprises a driving chip Q1 and a switching element Q2. The type of the driving chip Q1 is a P-MOS transistor. The G pin of the driving chip Q1 is connected with a switching element Q2, and the G pin of the driving chip Q1 is turned on or off through the switching element Q2, so that energy output or cut-off is achieved to make the heating pipe 20 to turn on or off heating. According to the received control signal the switching element Q2 controls the driving chip Q1 through turning on or off. Specifically, the above-mentioned switching element Q2 is a transistor or MOS transistor of PNP transistor, wherein the heating drive module 3 mentioned comprises a pull-up resistor R2 and a first current limiting resistor R5. The end of the pull-up resistor R2 is connected to BT+ and the other end is connected to the input pin (S-pin) of switching element Q2. The both ends of the first current limiting resistor R5 are respectively connected to the control pin (G-pin) of the driver chip Q1 and the input pin (S-pin) of switching element Q2.

The detection module 2 comprises a sampling resistor R1. The end of the sampling resistor R1 is connected to the output pin (ie, D-pole) of the driver chip Q1 and the other end is connected to the heating pipe 20.

According to the characteristics of the P-MOS transistor, when the voltage Vgs between the gate and the source is smaller than a certain value, that is 0.8v to 2.0v, the driving chip Q1 will be turned on, and vice versa will be turned off.

As shown in FIG. 5, when HOT_1 is configured to output a low level, the switching element Q2 is turned off, and the control pin (G pin) of the driving chip Q1 is at a high level, that is, the conduction between the input pin (S pole) and the output pin (D pin) of the driving chip Q1 is turned off; and the driving chip Q1 stops the work, the driving chip Q1 does not provide a working current to the heating conductor, and the heating pipe 20 does not work. When the HOT_1 is high level, the switching element Q2 is turned on, and the control pin (G pin) of the driving chip Q1 is low level, that is, the input (S pin) and the output (D pin) of the driving chip Q1 are turned on, and the driving chip Q1 provides a working current to the heating pipe 20, and the heating pipe 20 works.

As shown in Fig.4, The HOT_1 control signal is output from the control chip U4 of the control unit.

As shown in Fig.5, in addition, the detection module 2 comprises a current sampling chip U1, and the sampling resistor R1 is a current sampling resistor. The current flowing through the heating pipe 20 is equal to the current flowing through the sampling resistor R1. When the current flows through the sampling resistor R1, and the voltage is generated at both ends of the sampling resistor R1. Because the resistance of the sampling resistor R1 is relatively small, the voltage across the sampling resistor R1 is also relatively small, and after the voltage is amplified by the current amplifier several times, the voltage is converted into a suitable AD sampling voltage, which is collected by the PA4 pin of the control chip U4.The current sampling resistor requires a small resistance and the accuracy is controlled at 1%.

In addition, Further, the detection module 2 comprises a first voltage divider resistor R4 and a second voltage divider resistor R6. The detection module 2 collects the voltage of the heating pipe 20 by the first voltage divider resistor R4 and the second voltage divider resistor R6, and the voltage is provided to the control chip U4.

The number of the above heating units may be one, two or three; in other embodiments, the number of the above heating units is four or more, and the heating unit is arranged around the tobacco so as to uniformly heat the tobacco, and the release of cigarette smoke is more uniform.

Specifically, the above-mentioned control chip U4 has a PWM output port. According to the size of the airflow the mentioned control chip U4 can adjust the PWM duty cycle, thereby the control chip U4 adjusts the heating curve of the heating pipe 20.

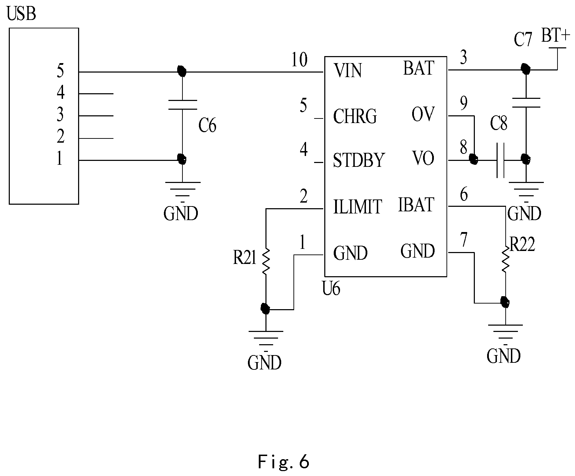

As shown in FIG. 6, in other embodiments, the infrared temperature control system comprises a charging unit 7, which is configured to charge for the power supply unit 8 connected to it according to external power input; and the power supply unit 8 is configured to provide a power for the control unit. In this embodiment, the power supply unit 8 is a combination of a battery, and the power supply unit 8 also provides energy for the operation of the electric heating device.

The charging unit 7 comprises a charging management chip U6 and a USB interface connected to the charging management chip U6. The type of the charging management chip U6 is LP28021.

In addition, in other embodiments, the above-mentioned infrared temperature control system further comprises a display unit 5 is configured to displa the current state of the electric heating device under the control of the control unit.

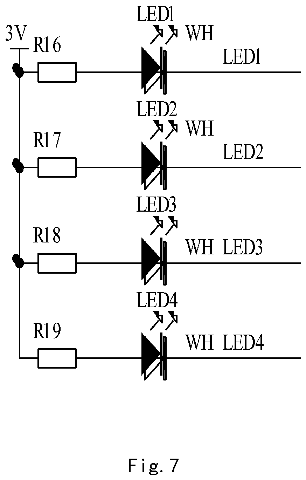

In this embodiment, as shown in FIG. 7, the above-mentioned display unit 5 is a plurality of LED lamps connected in parallel; in other embodiments, the above-mentioned display unit 5 may also be a display screen.

In other embodiments, the above-mentioned infrared temperature control system further comprises a switch unit 6, which is used for starting and closing the electric heating device to form an input signal of the control unit.

As shown in FIG. 4, the above-described switch unit 6 is a button SW1. In other embodiments, the above-mentioned switch unit 6 may also be other single-pole switches or the like.

After the electric heating device is turned on by pressing the button SW1. When the heating is started and the preset temperature is reached, the temperature of the heating pipe 20 will be used to adjust the output power to reach the constant temperature effect. The signal generated by the size of the air flow will not be the same. These signals are amplified, and the control chip U4 of the electric heating device controls the heating drive module 3 to achieve the adjustment of the heating rate.

Preferably, the number of the detection holes 21 is two, and the number of the infrared detection units is also two. The detection probes 121 corresponding to each infrared sensor 12 are respectively disposed in the two detection holes 21 of the heating pipe 20. When two infrared detection units respectively obtain detection signals through the two detection probes 121, it can be determined that the cigarette inserted into the heating pipe 20 is a long cigarette, and if the detection probe 121 placed at the lower end of the heating pipe 20 obtains a detection signal, it can be determined that the cigarette inserted into the heating pipe 20 is a short cigarette. If no detection signal is obtained by the two infrared detection units, it can be show that no cigarette is inserted into the heating pipe 20.

In other embodiments, if the number of detection holes 21 is three or more, the number of infrared detection units is the same as the number of detection holes 21, and the detection probes 121 are respectively disposed in the detection holes 21 of the heating pipe 20. For detecting different lengths of cigarettes, the more the number of detection holes 21, the more accurate the accuracy of detecting different lengths of cigarettes.

The invention also provides the infrared temperature control method of the electronic heating device, comprises:
the infrared detection unit emits an infrared signal through the detection hole of the heating pipe, and the infrared detection unit detects the condition in the heating pipe to determine whether a cigarette is inserted into the heating pipe and forms an input signal;
the control unit receives the input signal, and the control unit outputs the adjustment signal to the heating unit according to the input signal; and
the heating unit adjusts the heating temperature of the heating pipe 20 according to the adjustment signal.

After the cigarette is inserted into the heating pipe 20, the infrared signal emitted by the infrared detection unit can detect the presence of the cigarette, and send an input signal to the control unit. The control unit sends an instruction to start the heating unit, and then the heating unit heats the heating pipe 20, and the baked the cigarette in the heating pipe 20 emit the cigarette gas.

The number of heating units may be two or more. These heating units are respectively disposed in different segment areas of the heating pipe, and each heating unit corresponds to an infrared detection unit .Correspondingly, these detection holes 21 are arranged along the same vertical line on the heating tube 20. In this way, the infrared detection unit can judge whether the cigarette placed in the heating pipe 20 is a long-smoke bombarding or a short-smocking tantalum cigarette according to the reflection, So that the control unit controls the heating unit at different positions to heat and to adjust the heating temperature.

## Claims

1. An infrared temperature control system of an electronic heating device comprising a control unit, an infrared detection unit, and a heating pipe with at least two detection holes, wherein:
the infrared detection unit is configured to emit a detection signal and is configured to detect the conditions of the heating pipe through the detection hole, and is further configured to judge whether there is a cigarette in the heating pipe and to form an input signal;
the control unit is configured to output an adjustment signal to the heating unit according to the received input signal; and
the heating unit is configured to start heating and configured to adjust the heating temperature of the heating pipe according to the adjustment signal.

2. The infrared temperature control system of claim 1, wherein the infrared detecting unit comprises an infrared sensor and a detection drive module, wherein:
the infrared sensor is configured to detect the conditions of the heating pipe through the detection hole, and is configured to form a detection signal; and
the detection drive module is configured to form an input signal according to the detection signal.

3. The infrared temperature control system of claim 2, wherein the detection drive module comprises a switch module connected to the receiving diode (D1) of the infrared sensor.

4. The infrared temperature control system of claim 3, wherein the switch module comprises a first switching element (Q6) and a second switching element (Q5), wherein the control pin of the first switching element (Q6) is connected to the anode of the receiving diode (D1), and the cathode of the receiving diode (D1) is connected to the external power source (BT+); and
the output pin of the first switching element (Q6) is connected to the control terminal of the second switching element (Q5); and
the output pin of the second switching element (Q5) is connected to the control unit.

5. The infrared temperature control system of any one of claims 1, 2 or 3, wherein the control unit comprises a control chip (U4).

6. The infrared temperature control system of claim 5, wherein the heating unit comprises a heating drive module and a detection module, wherein:
the heating drive module is configured to receive a control signal and is configured to drive an external heating conductor to heat; and
the detection module is configured to obtain the current and the voltage of the heating drive module during operation and is configured to feedback the signal to the control unit to adjust the heating temperature of the heating drive module.

7. The infrared temperature control system of claim 6, wherein the heating drive module comprises a driving chip (Q1) and a switching element (Q2); wherein
according to the received control signal the switching element (Q2) is configured to control the driving chip (Q1) by turning on or off, and the detecting module comprises a current sampling chip (U1).

8. The infrared temperature control system of any preceding claim, wherein the infrared temperature control system comprises a charging unit, and according to an external power source the charging unit is configured to charge the power supply units connected with it.

9. The infrared temperature control system of any preceding claim, wherein the infrared temperature control system comprises a display unit and the display unit is configured to display the current state of the electric heating device under the control of the control unit.

10. An infrared temperature control method using the electronic heating device of any preceding claim, comprising:
the infrared detection unit emitting an infrared signal to detect the internal condition of the heating pipe through the detection hole of the heating pipe, and determining whether a cigarette is inserted into the heating pipe and forming an input signal;
the control unit receiving the input signal and outputting the adjustment signal to the heating unit; and
the heating unit adjusting the heating temperature of the heating pipe according to the adjustment signal.
